# DEMANDE DE BREVET EUROPEEN

(11) **EP 4 389 057 A1**
(43) Date de publication de la demande: **26.06.2024**
(21) Numéro de dépôt: 23217464.9
(22) Date de dépôt: 18.12.2023
(51) Int. Cl.: A61B 90/00, A61B 34/20

(54) **STYLET POUR SYSTÈME DE NUMÉRISATION EN TROIS DIMENSIONS**

(30) Priorité: 19.12.2022 FR 2213876
(71) Demandeur: One Ortho, 69230 Saint-Genis-Laval (FR); Université Savoie Mont Blanc, 73000 Chambéry (FR)
(72) Inventeur: VACHER, Pierre, 73000 CHAMBERY (FR); ELMO KULANESAN, Christian, 73000 CHAMBERY (FR); CHARLEUX, Ludovic, 73000 CHAMBERY (FR); ROUX, Emile, 73000 CHAMBERY (FR); ALEPEE, Christophe, 69230 SAINT-GENIS-LAVAL (FR)
(74) Mandataire: Cabinet Laurent & Charras

(57) **Abrégé**

L'invention concerne un stylet pour système de numérisation en trois dimensions par palpage manuel et système de vision, présentant un palpeur et un localisateur comprenant plusieurs marqueurs (M) uniques et présentant chacun une normale d'orientation différente les unes par rapport aux autres ; caractérisé en ce que les marqueurs (M) sont en nombre, en position et en orientation pour qu'au moins deux marqueurs (M) soient lisibles par une caméra du système de vision, selon tout point de vue du localisateur en condition de palpage.

## Description

### Domaine technique

La présente invention se rapporte à la numérisation en trois dimensions, et plus particulièrement à la numérisation par palpage manuel à l'aide d'un stylet surmonté d'un localisateur passif.

### Art antérieur

Les marqueurs plans sont largement utilisés dans les systèmes de localisation spatiale par vision. Le principe de fonctionnement se décompose généralement en deux étapes principales :
- la détection du marqueur sur une image ; et
- l'estimation de la pose décrivant la position spatiale de ce marqueur.

Les marqueurs dédiés à la localisation spatiale sont majoritairement de couleur binaire (noir et blanc), de forme carrée (« ArUco » contenus dans la bibliothèque libre « OpenCV », « AprilTag » développés par l'université du Michigan) ou circulaire (« CCTag » contenus dans la bibliothèque libre éponyme). De tels marqueurs sont constitués d'une matrice permettant d'identifier les différents marqueurs du localisateur. Le déchiffrement de cette matrice est propre au type de marqueur employé.

Un marqueur plan peut être fixé sur un objet mobile afin de suivre les mouvements de ce dernier.

Dans le domaine de la navigation chirurgicale, ce marqueur est positionné sur un stylet à l'extrémité duquel a été fixé un palpeur. Ce stylet est utilisé afin de numériser en trois dimensions, manuellement, des surfaces osseuses quelconques. Il est observé par une caméra placée à environ 1 mètre de distance.

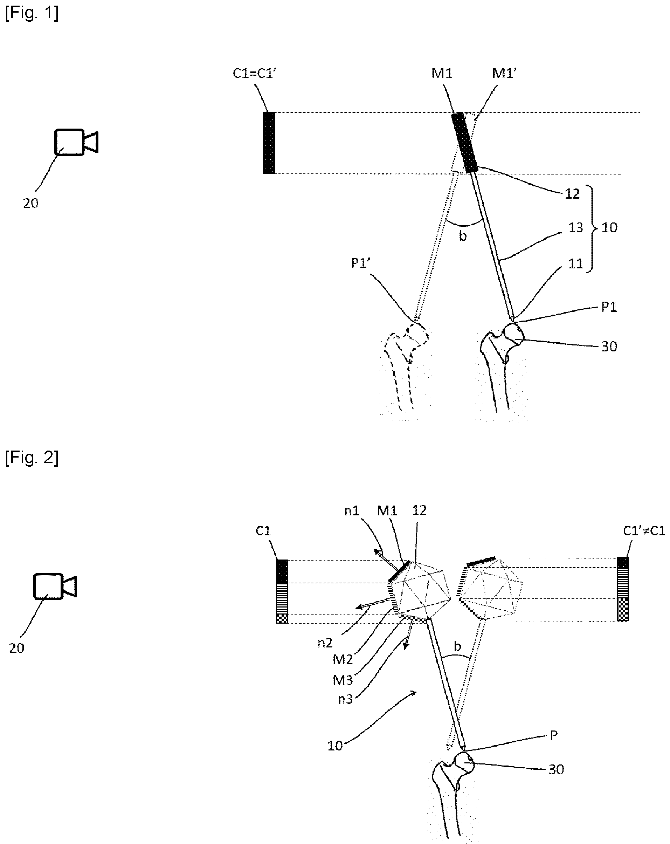

Un phénomène appelé « ambiguïté de pose » apparait pour certaines positions de ce marqueur. En référence à la figure 1, on voit que la caméra (20) peut capter une même image (C1, C1') correspondant à deux positions différentes du stylet, décalés d'un angle (b). Le système de numérisation ne peut pas détecter si le stylet (10) est en train de palper un premier point (P1) ou un autre point (P1'). En raison des incertitudes de localisation de ce marqueur, il devient alors compliqué, voire impossible, de définir par vision monoculaire la pose réelle du stylet.

De plus, un marqueur partiellement masqué, taché ou encore non plan (s'il est collé sur une surface non plane, ou s'il se décolle) perturbe très fortement la position spatiale retrouvée. Les incertitudes de mesures obtenues ne sont pas compatibles avec les exigences de la numérisation, et le stylet doit être remplacé.

### Exposé de l'invention

L'invention vise donc à pallier les inconvénients précités, et en particulier vise à proposer un stylet permettant d'éviter les ambiguïtés de pose.

L'invention vise également à proposer un stylet qui soit durable.

Enfin, l'invention vis à proposer un stylet qui soit plus facile d'utilisation pour le praticien.

À cet effet, il a été mis au point un stylet pour système de numérisation en trois dimensions par palpage manuel et système de vision présentant un palpeur et un localisateur comprenant plusieurs marqueurs uniques et présentant chacun une normale d'orientation différente les unes par rapport aux autres.

Selon l'invention, les marqueurs sont en nombre, en position et en orientation pour qu'au moins deux marqueurs soient lisibles par une caméra du système de vision, selon tout point de vue du localisateur en condition de palpage.

De cette manière, quelle que soit la position ou l'orientation du palpeur, on garantit que plusieurs marqueurs seront lisibles par la caméra, ce qui permet d'éviter les ambiguïtés de pose lorsqu'on utilise une caméra monoculaire. De plus, le praticien n'a pas à s'inquiéter d'orienter correctement stylet par rapport à la caméra : il est donc plus libre dans les mouvements lors du palpage, qui est facilité. Par ailleurs, la lecture de deux marqueurs au lieu d'un seul augmente la résolution de la lecture effectuée : la numérisation est plus précise.

Par « marqueurs uniques », on signifie que les marqueurs sont individuels, différents les uns des autres.

Par caméra, on entend tout dispositif de captation optique telle qu'un appareil photo, une caméra ou un capteur, configuré pour obtenir une image des marqueurs.

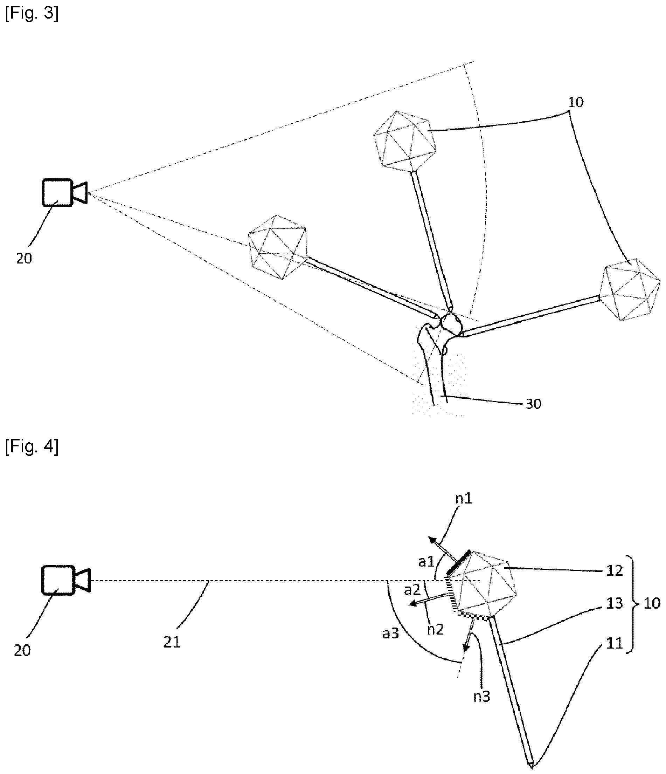

L'expression « selon tout point de vue du localisateur en condition de palpage » signifie toutes les dispositions et inclinaison raisonnablement prévisibles du localisateur lors de l'étape de palpage, sous réserve que l'image du localisateur soit nette (profondeur de champ de la caméra compatible, et pas de flou de bougé lors de l'acquisition). Des exemples de positions sont illustrés figure 3.

Le localisateur est passif, c'est à dire qu'il n'émet ni lumière, ni ondes radio destinées à être captées par le système pour que ce dernier détermine la position du localisateur.

Dans un mode de réalisation préféré, les marqueurs sont en nombre, en position et en orientation pour qu'au moins trois marqueurs soient lisibles par une caméra, quelle que soit la position du marqueur. Ce mode confère deux avantages distincts :
- l'information captée par la caméra est redondante car seulement deux marqueurs auraient été suffisants pour lever l'ambiguïté de pose. La redondance de l'information permet de rendre l'estimation de la pose du stylet encore plus précise, donc la numérisation en trois dimensions sera de meilleure qualité.
- la durabilité du stylet est augmentée, car si un marqueur devenait défaillant (soit à cause d'une salissure lors du palpage, soit à cause d'une détérioration par usure) alors le stylet reste fonctionnel car deux marqueurs sont suffisants pour éviter les ambiguïtés de pose
- la performance du stylet est augmentée car une image captée par la caméra est exploitable même si le localisateur est partiellement masqué (par le chirurgien ou par un objet quelconque) : il suffit que deux marqueurs soient visibles.

Afin de garantir une bonne lisibilité des marqueurs, les marqueurs sont des codes à deux dimensions, tel qu'un code à barres à 2 dimensions.

Toujours pour garantir une bonne lisibilité, pour un point de vue donné, les normales des trois marqueurs visibles présentent une inclinaison inférieure à 75° par rapport à l'axe optique du point de vue, et de préférence inférieure à 60°.

Pour que la résolution de la numérisation soit bonne, le palpeur présente une géométrie sphérique de rayon compris entre 0,2mm et 2mm, et de préférence égal à 1mm.

Dans un autre mode de réalisation, le palpeur présente une géométrie sous forme de pointe, pour palper certaines zones articulaires en traversant la zone cartilagineuse, afin de toucher directement l'os.

Afin d'assurer une distribution des marqueurs qui soit régulière, les normales des marqueurs sont chacune parallèle à la normale d'une face d'un polyèdre uniforme tel qu'un dodécaèdre, un icosaèdre, un cube tronqué ou un octaèdre tronqué. Une distribution régulière permet d'éviter que certaines orientations du localisateur soient moins performantes que d'autres.

Dans un mode, le localisateur présente une géométrie irrégulière. Ce mode ne présente pas les avantages précités, néanmoins la conception et la fabrication du localisateur s'en trouvent facilitées car les contraintes géométriques sont moindres.

Avantageusement, les marqueurs mesurent entre 8 et 20 mm, de préférence entre 10mm et 15mm, et encore plus préférentiellement mesurent 12mm. Par « dimension », on entend la longueur des côtés d'un marqueur carré, ou le diamètre d'un marqueur circulaire. Ces dimensions permettent d'avoir un bon compromis entre l'encombrement du localisateur et la lisibilité des marqueurs, lorsque la caméra est placée à une distance d'environ 1 m du stylet lors du palpage.

L'invention concerne également un système de numérisation en trois dimensions par palpage manuel et système de vision monoculaire comprenant :
- un stylet selon les caractéristiques précitées ;
- une caméra configurée pour acquérir une image du localisateur lors du palpage ;
- un ordinateur exécutant un programme d'ordinateur programmé pour :
   - identifier les marqueurs visibles sur l'image acquise ;
   - déterminer la position du localisateur par rapport à la caméra ;
   - déterminer la position du palpeur par rapport au localisateur ;
   - reconstruire une géométrie de la surface palpée à partir de plusieurs positions déterminées du palpeur. Un tel système permet de bénéficier des avantages précités du stylet. En particulier, le stylet selon l'invention permet d'utiliser une caméra monoculaire, ce qui réduit le coût du système, et qui libère le champ opératoire car il n'y a qu'un seul champ de vision de caméra à dégager. La précision obtenue de la localisation est néanmoins équivalente et parfois supérieure à certains systèmes stéréoscopiques de l'art antérieur.

L'invention concerne également un procédé d'étalonnage d'un tel système de vision, et comprenant les étapes suivantes :
- la caméra acquiert plusieurs images du localisateur selon différents points de vue ;
- le programme d'ordinateur est programmé pour :
   - identifier les marqueurs visibles sur chaque image acquise ;
   - modéliser en trois dimensions les positions relatives des marqueurs sur le localisateur ;
la position du palpeur par rapport au localisateur étant prédéterminée. Un tel procédé permet de pouvoir utiliser facilement un nouveau stylet avec un système préexistant. La position du palpeur par rapport au localisateur peut être déterminée au préalable, par palpage d'un étalon de géométrie connue.

De manière à réduire les ressources de mémoire nécessaire pour l'ordinateur, le programme d'ordinateur est programmé pour évaluer la lisibilité d'un marqueur, de manière à soit prendre en compte le marqueur lors des acquisitions (lors du palpage) si sa lisibilité est suffisante, soit l'ignorer si sa lisibilité est insuffisante. L'ignorer permet d'éviter des erreurs d'identification du marqueur et des ambiguïtés de pose. L'ignorer permet également d'économiser des ressources de calcul, et les temps de réponse du programme ordinateur s'en trouvent améliorés.

Avantageusement, le programme d'ordinateur est programmé pour émettre une alerte lorsque la disposition des marqueurs illisibles est telle qu'il n'y a pas au moins deux marqueurs lisibles selon tout point de vue du localisateur. Ce procédé permet de vérifier si un stylet est toujours fonctionnel, malgré son vieillissement ou les détériorations qu'il aurait pu subir.

### Description des figures

[Fig.1] est un schéma illustrant les ambiguïtés de pose pouvant survenir avec les stylets de l'art antérieur.
[Fig.2] est un schéma illustrant un stylet selon l'invention.
[Fig.3] est un schéma illustrant différentes positions que peut adopter un stylet lors d'un palpage.
[Fig.4] est un schéma illustrant les inclinaisons de normales des marqueurs par rapport au point de vue de la caméra.
[Fig.5] est un schéma illustrant un localisateur selon le point de vue de la caméra.
[Fig.6] est un schéma illustrant une image captée par la caméra.
[Fig.7] est un schéma illustrant une image similaire à celle de la figure 6, sur laquelle un marqueur n'est pas lisible.
[Fig.8] est un schéma illustrant un stylet dont le localisateur est de forme irrégulière.

### Description détaillée

En référence aux figures 2 à 8, l'invention se rapporte :
- à un stylet (10) pour palpage manuel permettant d'effectuer une numérisation en trois dimensions ;
- à un système de numérisation par palpage manuel ; et
- à un procédé d'étalonnage d'un tel système.

En référence particulière aux figures 2 et 3, un stylet (10) pour numérisation par palpage manuel comprend :
- un palpeur (11) destiné à être positionné sur une surface à numériser d'un objet (30), par exemple une partie d'une articulation ;
- un localisateur (12) comprenant des marqueurs (M), tels que des codes à barres de dimensions, destinés à être lu par une caméra (20) ;
- une tige (13) reliant le palpeur (11) au localisateur (12).

En fonction de la disposition relative des marqueurs (M), un programme ordinateur est programmé pour déterminer la position du localisateur (12), et par conséquent la position du point sur lequel est posé le palpeur (11). La détermination d'un nombre suffisant de points scannés par ce stylet permet de numériser la surface de l'objet (30).

De manière à lever toute ambiguïté de pose, le localisateur (12) présente, au moins deux marqueurs (M) lisibles par la caméra (20), quelle que soit sa position lors du palpage. Pour cela, le localisateur (12) présente des marqueurs (M) en nombre, en position, et en localisation adaptés.

Sur la figure 2, on reproduit le même écart d'angle (b) entre les inclinaisons du stylet (10). Une schématisation des images captées par la caméra (20) montre que la captation (C1) correspondant à la pose sur le point (P) ne peut pas être confondue avec une autre captation (C1') correspondant à une autre inclinaison.

Par conséquent, le chirurgien n'est plus obligé d'orienter le localisateur (12) pour s'assurer que les marqueurs (M) sont bien face à la caméra (20) lors de la pose du stylet (10). Il est beaucoup plus libre de ses mouvements, ce qui facilite le palpage.

La figure 3 illustre de manière schématique différentes positions qu'est susceptible d'adopter un stylet (10) lors d'un palpage. Les inclinaisons du stylet (10) peuvent être diverses et variées. Par rapport à la caméra (20), il peut y avoir un angle mort disposé derrière l'objet (30) à palper. Dans ce cas, la longueur de la tige (13) du stylet (10), généralement comprise entre 10 cm et 25 cm, permet de palper cette zone tout en disposant le localisateur (12) dans le champ de la caméra (20).

En référence aux figures 2, 4 et 5, on a illustré les orientations que présentent les marqueurs (M) par rapport à la caméra (20). Le localisateur (12) présente une multitude de marqueurs. Bien que plusieurs marqueurs (M) soient visibles par la caméra (20), tous ne sont pas lisibles par celle-ci.

En effet, comme illustré figure 4, si un marqueur (M) présente une inclinaison trop importante par rapport à la caméra (20), celui-ci n'est pas lisible. Sur la figure 4 :
- les marqueurs (M) M1, M2 et M3 sont correctement orientés par rapport à la caméra (20), de sorte que leur lisibilité est possible ;
- les marqueurs (M) M4, M5 et M6 sont trop inclinés et ne sont pas lisibles.

La mesure de l'inclinaison des marqueurs (M) se fait entre l'axe optique (21) de la caméra (20), et la normale (n) des marqueurs. Par exemple, les normales (n1, n2, n3) respectivement des marqueurs (M1, M2, M3) sont représentées sur les figures 2 et 4. Les angles correspondant (a1, a2, a3) sont représentés sur la figure 4.

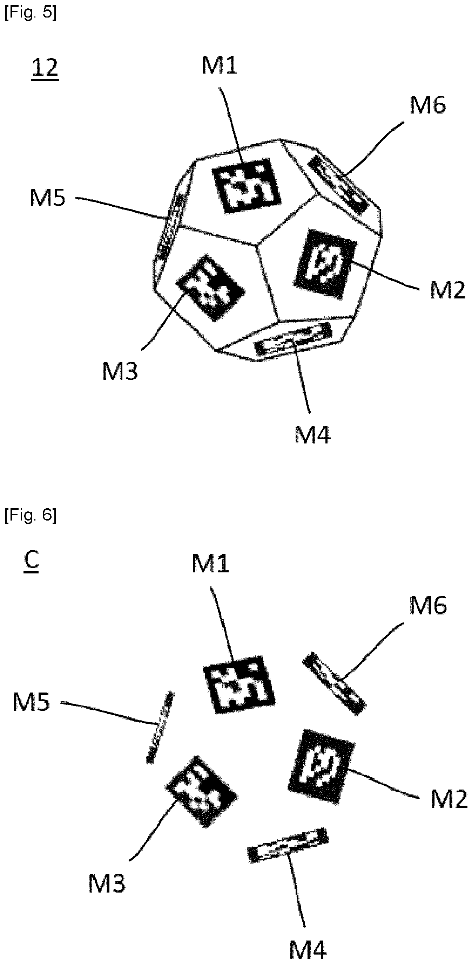

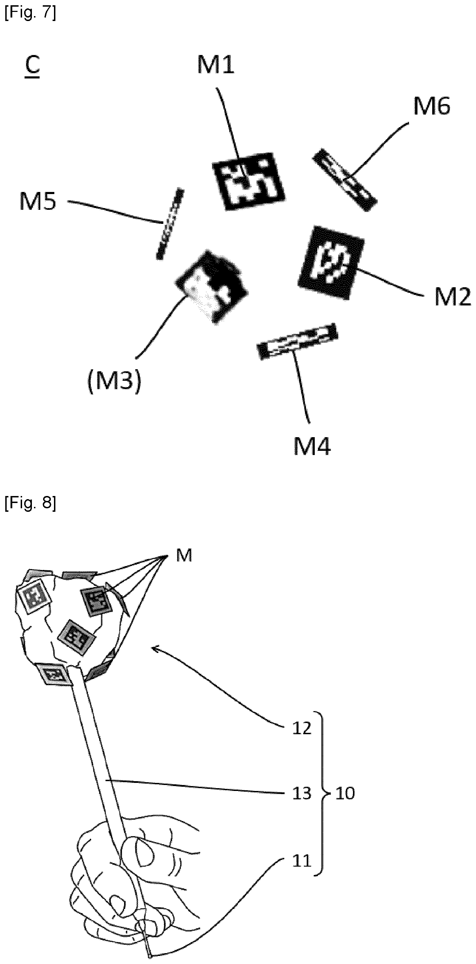

La figure 6 illustre une captation (C) effectuée par la caméra (20), et permet d'illustrer que le problème posé est résolu par l'orientation des marqueurs (M), pas nécessairement par la géométrie en tant que telle du localisateur (12) :
- le localisateur (12) peut être un solide convexe, et en particulier un polyèdre uniforme convexe tel que le dodécaèdre de la figure 4, un icosaèdre, un cube tronqué ou un octaèdre tronqué ;
- le localisateur (12) peut être un solide non convexe, par exemple sous la forme d'une étoile portant à chacune de ses extrémités un marqueur (M) : une étoile régulière à douze branches fournirait la même captation que celle d'un dodécaèdre ;
- le localisateur (12) peur être un solide irrégulier, tel qu'illustré figure 8.

En pratique, un polyèdre uniforme convexe permet d'obtenir une distribution régulière des marqueurs (M), de sorte que la performance de lecture est la même, quelle que soit l'orientation du stylet (10) lors du palpage.

Un solide convexe ne présente pas de zones de rétention : le nettoyage du stylet (10) est facilité, ce qui est préférable dans le domaine médical, et en particulier celui de la chirurgie.

La figure 7 illustre une captation (C) sur laquelle un marqueur (M3) est détérioré. En fonction des dégradations, un marqueur (M) détérioré peut ne pas être lu par le programme, ou encore être mal lu par le programme, c'est-à-dire fournir :
- une bonne identification du marqueur (M), mais dans une mauvaise orientation ; ou
- une mauvaise identification du marqueur (confusion entre deux marqueurs).

Dans le cas d'une mauvaise lecture, le programme d'ordinateur sera induit en erreur et la détermination de la pose pourrait conduire à une ambigüité.

Afin de se prémunir de tels inconvénients, un procédé d'étalonnage du système de numérisation comprend une étape d'évaluation de la lisibilité des marqueurs. Lors de cette étape, le programme d'ordinateur est programmé pour évaluer la lisibilité de chaque marqueur (M), de manière à :
- soit prendre en compte le marqueur (M) lors des acquisitions, si sa lisibilité est suffisante ;
- soit ignorer le marqueur (M) si sa lisibilité est insuffisante.

Une fois que le système est étalonné selon cette méthode, le programme d'ordinateur est programmé pour ne pas prendre en compte le marqueur (M) défectueux. Cela signifie que sur les images captées par la caméra (20), le programme ordinateur est programmé pour ignorer les données susceptibles de correspondre à celle du marqueur (M) défectueux. Ces données sont identifiées par leur position par rapport aux autres marqueurs (M) encore lisibles, par exemple les marqueurs (M) M1 et M2 sur la figure 6. Les risques d'ambigüités de pose sont supprimés.

L'invention concerne également un procédé d'étalonnage d'un système de numérisation en trois dimensions, permettant de pouvoir utiliser un nouveau stylet (10) avec un système préexistant. Cette séquence d'étalonnage comprend les étapes suivantes :
- la caméra (20) acquiert plusieurs images du localisateur (12) selon différents points de vue ;
- le programme d'ordinateur est programmé pour :
   - identifier les marqueurs (M) visibles sur chaque image acquise ;
   - modéliser en trois dimensions les positions relatives des marqueurs (M) sur le localisateur (12).

Bien entendu, il est nécessaire d'acquérir suffisamment d'images pour que chaque marqueur (M) ait été visualisé au moins une fois par la caméra (20).

Ainsi, pour chaque image captée lors du palpage, le programme d'ordinateur sera apte à identifier les au moins deux marqueurs (M) lisibles, puis à déterminer la position (y compris l'orientation) du localisateur (12).

La position du palpeur (11) par rapport au localisateur (12) étant prédéterminée dans le programme, la position du palpeur (11) pourra donc être déterminée elle aussi.

Au surplus, le procédé d'étalonnage peut comprendre une étape de vérification de la conformité du localisateur (12). Pour cela, le programme d'ordinateur est programmé pour émettre une alerte lorsque la disposition des marqueurs (M) illisibles est telle qu'il n'y a pas au moins deux marqueurs (M) lisibles selon tout point de vue du localisateur (12). De cette manière, il est possible de vérifier qu'un stylet (10) est toujours fonctionnel (absence d'alerte), ou de remplacer ou de réparer le stylet (10) défectueux (en cas d'alerte).

Un stylet (10) selon l'invention permet d'obtenir :
- la position du palpeur (11) avec une incertitude inférieure à 1 mm3 ; et
- l'orientation de la tige (13) du stylet (10) avec une incertitude inférieure à 1°.

Le stylet (10) est localisable quelle que soit sa position dans le champ d'observation, du fait de la multitude et la disposition des marqueurs (M) sur le localisateur (12).

Le stylet (10) est compatible aussi bien avec des systèmes comprenant plusieurs caméras (20), qu'avec des systèmes monoculaires.

Par ailleurs, le stylet (10) et le localisateur (12) peuvent être conformés différemment des exemples donnés sans sortir du cadre de l'invention, qui est défini par les revendications.

En particulier, les marqueurs (M) peuvent être conformés différemment d'un code à barre à deux dimensions, peuvent être de tout type adapté à la présente application. Les marqueurs (M) peuvent être de différentes couleurs.

En outre, les caractéristiques techniques des différents modes de réalisation et variantes mentionnés ci-dessus peuvent être, en totalité ou pour certaines d'entre elles, combinées entre elles. Ainsi, le stylet (10) et le localisateur (12) peuvent être adaptés en termes de coûts, de fonctionnalités et de performance.

## Revendications

1. Stylet (10) pour système de numérisation en trois dimensions par palpage manuel et système de vision, présentant un palpeur (11) et un localisateur (12) comprenant plusieurs marqueurs (M) uniques et présentant chacun une normale d'orientation différente les unes par rapport aux autres ;
***caractérisé en* ce *que*** les marqueurs (M) sont en nombre, en position et en orientation pour qu'au moins deux marqueurs (M) soient lisibles par une caméra (20) du système de vision, selon tout point de vue du localisateur (12) en condition de palpage.

2. Stylet (10) selon la revendication 1, ***caractérisé en* ce *que*** les marqueurs (M) sont en nombre, en position et en orientation pour qu'au moins trois marqueurs (M) soient lisibles par une caméra (20) du système de vision, selon tout point de vue du localisateur (12) en condition de palpage.

3. Stylet (10) selon l'une des revendications précédentes, ***caractérisé en ce que*** les marqueurs (M) sont des codes à 2 dimensions, tel qu'un code à barres à 2 dimensions, **et *en ce que*** pour un point de vue donné, les normales des trois marqueurs (M) visibles présentent une inclinaison inférieure à 75° par rapport à l'axe optique du point de vue, et de préférence inférieure à 60°.

4. Stylet (10) selon l'une des revendications précédentes, ***caractérisé en ce que*** le palpeur (11) présente une géométrie sphérique de rayon compris entre 0,2mm et 2mm, et de préférence égal à 1mm.

5. Stylet (10) selon l'une des revendications précédentes, ***caractérisé en ce que*** les normales des marqueurs (M) sont chacune parallèle à la normale d'une face d'un polyèdre uniforme tel qu'un dodécaèdre, un icosaèdre, un cube tronqué ou un octaèdre tronqué.

6. Stylet (10) selon l'une des revendications 1 à 4, ***caractérisé en ce que*** le localisateur (12) présente une géométrie irrégulière.

7. Stylet (10) selon l'une des revendications précédentes, ***caractérisé en ce que*** les marqueurs (M) mesurent entre 8 et 20 mm, de préférence entre 10mm et 15mm, et encore plus préférentiellement mesurent 12mm.

8. Système de numérisation en trois dimensions par palpage manuel et système de vision monoculaire comprenant :
- un stylet (10) selon l'une des revendications précédentes,
- une caméra (20) configurée pour acquérir une image du localisateur (12) lors du palpage ;
- un ordinateur exécutant un programme d'ordinateur programmé pour :
- identifier les marqueurs (M) visibles sur l'image acquise ;
- déterminer la position du localisateur (12) par rapport à la caméra (20) ;
- déterminer la position du palpeur (11) par rapport au localisateur (12) ;
- reconstruire une géométrie de la surface palpée à partir des positions du palpeur (11) déterminées.

9. Procédé d'étalonnage d'un système selon la revendication 8, ***caractérisé en ce qu***'il comprend les étapes suivantes :
- la caméra (20) acquiert plusieurs images du localisateur (12) selon différents points de vue ;
- le programme d'ordinateur est programmé pour :
- identifier les marqueurs (M) visibles sur chaque image acquise ;
- modéliser en trois dimensions les positions relatives des marqueurs (M) sur le localisateur (12) ;
la position du palpeur (11) par rapport au localisateur (12) étant prédéterminée.

10. Procédé d'étalonnage selon la revendication 9, ***caractérisé en ce que*** le programme d'ordinateur est programmé pour évaluer la lisibilité d'un marqueur (M), de manière à :
- soit prendre en compte le marqueur (M) lors des acquisitions, si sa lisibilité est suffisante ;
- soit ignorer le marqueur (M) si sa lisibilité est insuffisante.

11. Procédé d'étalonnage selon l'une des revendications 9 ou 10, ***caractérisé en ce que*** le programme d'ordinateur est programmé pour émettre une alerte lorsque la disposition des marqueurs (M) illisibles est telle qu'il n'y a pas au moins deux marqueurs (M) lisibles selon tout point de vue du localisateur (12).
